# EUROPEAN PATENT APPLICATION

(11) **EP 4 800 579 A1**
(43) Date of publication of application: **02.09.2026**
(21) Application number: 25160402.1
(22) Date of filing: 26.02.2025
(51) Int. Cl.: G06F 18/2131, G06V 10/82, G06V 20/69

(54) **METHOD AND SYSTEM FOR CLASSIFYING HISTOLOGICAL IMAGES**

(71) Applicant: Owkin France SAS, 75009 Paris (FR)
(72) Inventor: PIGNET, Arthur, 75009 PARIS (FR); OLIVIER, Antoine, 75009 PARIS (FR); ROBIN, Geneviève, 75009 PARIS (FR); KLEIN, John, 75009 PARIS (FR)
(74) Representative: Plasseraud IP

(57) **Abstract**

Provided is computer-implemented method for classifying an input image obtained in a first environment. The method comprises tiling the input image into a set of tiles, generating first tile scores for the set of tiles using a machine learning (ML) model, applying a transport map to the generated first tile scores to obtain second tile scores, using the second tile scores in the ML model to generate an image score for the input image, and comparing the image score to a threshold to classify the input image. The threshold is with respect to the second environment.

## Description

### TECHNICAL FIELD

The present invention relates to the field of image classification. In particular, the present invention relates to a method and system for classifying images assigned to respective image scores.

### BACKGROUND

In modern clinical practice, histological image analysis and classification is an important step for many diagnoses, especially in oncology. In order to improve the image classification and therefore the diagnostic accuracy, digital pathology, such as computational pathology (Cpath), plays a crucial part, wherein advanced computer vision and machine learning technologies, such as deep learning (DL) models, are integrated into diagnostic workflows to analyse histological images, such as whole slide images (WSI) (see also Liu, Y. et al.: Artificial Intelligence-Based Breast Cancer Nodal Metastasis Detection: Insights Into the Black Box for Pathologists. Archives of Pathology & Laboratory Medicine 143(7), 859-868 (Jul 2019). https://doi.org/10.5858/arpa.2018-0147-OA; Krithiga, R., Geetha, P.: Breast Cancer Detection, Segmentation and Classification on Histopathology Images Analysis: A Systematic Review. Archives of Computational Methods in Engineering 28(4), 2607-2619 (Jun 2021). https://doi.org/10.1007/s11831-020-09470-w; or Saillard, C. et al.: Validation of MSIntuit as an AI-based pre-screening tool for MSI detection from colorectal cancer histology slides. Nature Communications 14(1), 6695 (Nov 2023). https://doi.org/10.1038/s41467-023-42453-6).

However, the deployment of machine learning technologies in a clinical practice is still limited by their ability to generalize well beyond the training context, with inherent cohort variability due to the use of different scanners, tissue preparation, staining and labelling protocols, for example (see also Stacke, K. et al.: Measuring Domain Shift for Deep Learning in Histopathology. IEEE Journal of Biomedical and Health Informatics 25(2), 325-336 (Feb 2021). https://doi.org/10.1109/JBHI.2020.3032060). To overcome this limitation, recent works have relied on domain generalization (DG) techniques, which are designed to increase the robustness of predictive models to distribution shifts between training and evaluation cohorts (see also Farahani, A. et al.: A Brief Review of Domain Adaptation. In: Advances in Data Science and Information Engineering, pp. 877-894. Springer International Publishing (2021)). In the specific context of Cpath, DG techniques include data augmentation (see also Tellez, D. et al.: Quantifying the effects of data augmentation and stain color normalization in convolutional neural networks for computational pathology. Medical Image Analysis 58, 101544 (Dec 2019). https://doi.org/10.1016/j.media.2019.101544; Chen, R.J. et al.: Synthetic data in machine learning for medicine and healthcare. Nature Biomedical Engineering 5(6), 493-497 (Jun 2021). https://doi.org/10.1038/s41551-021-00751-8; or Jarkman, S. et al.: Generalization of Deep Learning in Digital Pathology: Experience in Breast Cancer Metastasis Detection. Cancers 14(21), 5424 (Nov 2022). https://doi.org/10.3390/cancers14215424), generalizable representation learning through foundational models (see also Zhang, Y. et al.: Text-Guided Foundation Model Adaptation for Pathological Image Classification. In: Medical Image Computing and Computer Assisted Intervention - MICCAI 2023, vol. 14224, pp. 272-282 (2023); Chen, R.J. et al.: Towards a general-purpose foundation model for computational pathology. Nature Medicine 30(3), 850-862 (Mar 2024). https://doi.org/10.1038/s41591-024-02857-3; or Filiot, A. et al.: Phikon-v2, A large and public feature extractor for biomarker prediction (2024). https://doi.org/10.48550/ARXIV.2409.09173), and DG-specific training algorithms (see also Gulrajani, I., Lopez-Paz, D.: In search of lost domain generalization. In: International Conference on Learning Representations (2021)).

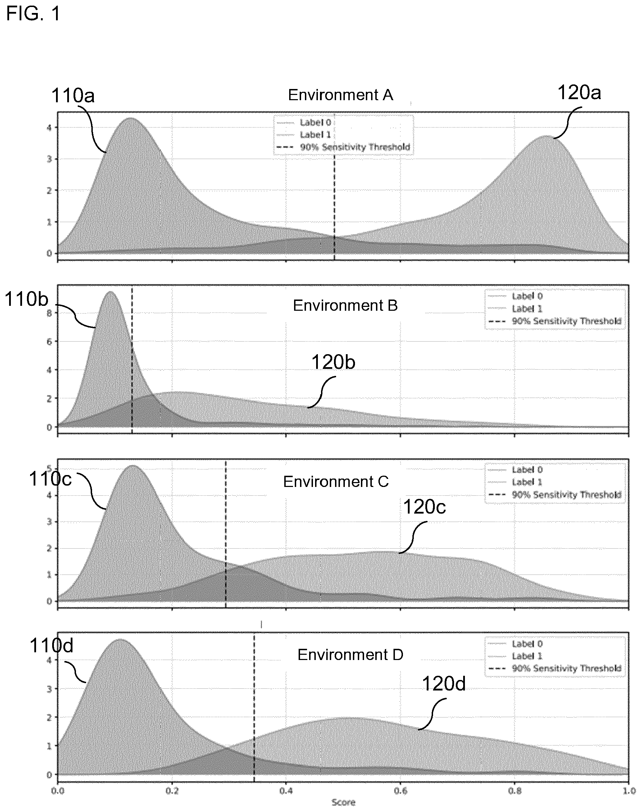

The performance of such DG models is usually evaluated by reporting the Area Under the ROC Curve (AUC) ("ROC" stands for "receiver operating characteristic"), which is insufficient to quantify the generalization capacity of DL models in a clinical context (see also Kleppe, A.: Area under the curve may hide poor generalisation to external datasets. ESMO Open 7(2), 100429 (Apr 2022). https://doi.org/10.1016/j.esmoop.2022.100429). Indeed, real-world examples show that models can exhibit good generalization properties in terms of AUC, while failing dramatically to generalize in terms of, for example, sensitivity and/or specificity, standard metrics considered for clinical deployment (see also Echle, A. et al.: Artificial intelligence for detection of microsatellite instability in colorectal cancer-a multicentric analysis of a pre-screening tool for clinical application. ESMO Open 7(2), 100400 (Apr 2022). https://doi.org/10.1016/j.esmoop.2022.100400). This can be explained by the fact that, in binary classification tasks, DL models usually output continuous scores which are translated into binary labels using a threshold controlling the sensitivity and specificity trade-off, sometimes called operating point. Even when models have been shown to generalize well in terms of AUC, the distribution of the continuous prediction scores might vary between cohorts, sites, and scanners (see also Roschewitz, M. et al.: Automatic correction of performance drift under acquisition shift in medical image classification. Nature Communications 14(1), 6608 (Oct 2023). https://doi.org/10.1038/s41467-023-42396-y), impairing the ability of the model to yield consistent sensitivity and specificity across clinical settings (see FIG.1 described below). To solve this issue, existing works have relied on calibration procedures aiming to adjust prediction score distributions.

As already indicated above, techniques are applied in digital pathology to provide a binary classification of the histological images, wherein image scores are assigned to each histological image. These image scores are usually compared with a threshold in order to determine whether biomarkers, gene mutations, or tumour cells, for example, are present in the respective histological images. This enables to provide a diagnosis for patients from whom the histological images have been taken, wherein the patients can be easily diagnosed as being healthy or having a disease.

However, setting a threshold, also called threshold calibration, is a laborious and tedious step that needs to be repeated for every scenario, such as every environment. The environment may be a medical centre environment, such as an hospital environment, or a specific patient cohort. The hospital environment may be a hospital. Thus, the problem of threshold calibration for binary classification is a very active research field in machine learning.

For example, a conventional method for threshold calibration is during a calibration phase in which positive or negative histological images are sampled to estimate the sensitivity or specificity of a classification model. Based on a sensitivity or specificity target, an adequate threshold may be set. This is for instance shown in FIG. 1 which illustrates a state-of-the art threshold calibration by way of example.

In FIG. 1, density plots for four different scenarios A to D, also called environments A to D, with similar AUC (AUC of scenario A: 94.7%, AUC of scenario B: 89.8%, AUC of scenario C: 91.7%, AUC of scenario D: 94.7%) are illustrated, wherein the x-axis represents image scores between 0 and 1 and the y-axis represents the density of each image score. Thus, the density plots show the probability density function of the image scores, represented by a smooth curve, to predict microsatellite instability (MSI) from WSI in colorectal cancer. For example, the environments A to D are different patient cohorts, or different medical centre environments, such as hospital environments, wherein the medical centre environments may differ in patient demographics, measurement equipment, etc.. The hospital environments may be hospitals. The term "patient cohort" is used in medical research to define groups of individuals with common characteristics, such as social and health factors. It is noted that the MSI is a key biomarker in colorectal cancer, whose early recognition may benefit patients by guiding them towards specific therapies, in particular colorectal cancer.

For environment A, two different density plots 110a and 120a may be generated. According to an example, the density plot 110a represents the probability density function of the image scores generated for histological images obtained in the environment A and labelled with a binary value of "0", whereas the density plot 120a represents the probability density function of the image scores generated for histological images obtained in the environment A and labelled with a binary value of "1". The label "0" may be assigned to histological images for which specific biomarkers, gene mutations, or tumour cells, for example, are not present (e.g. MSI is not present). The label "1" may be assigned to histological images for which specific biomarkers, gene mutations, or tumour cells, for example, are present (e.g. MSI is present). Thus, the density plots 110a and 120a illustrate the probability density function of the image scores for each label separately.

A threshold, also called decision threshold, for binary classification may be needed, wherein high sensitivity should be ensured. If a pre-specified sensitivity target of 90.0% should be reached, for example, a threshold of value 0.5 may be set for environment A (see the dashed line in FIG. 1 representing the threshold). This means that a histological image having an image score of 0.5 or less may now be assigned with a label "0", whereas a histological image having an image score of more than 0.5 may be assigned with a label "1".

While this approach for threshold calibration may work well in practice when there is access to positive samples, such as histological images being assigned with label "1", it can become very problematic when positive samples have a low prevalence in the patient cohort, for example. For instance, if a low prevalence of approximately 1.6% of positive patients, i.e. patients from which histological images with label "1" can be obtained, is given (which may be the case for MSI for small intestine, wherein the label "1" indicates presence of MSI), scanning of a large number of patients, such as 2000 or more, is required. Thus, threshold calibration may require a large amount of resources in order to be able to scan a great number of patients and obtain a large number of histological images.

Furthermore, a single threshold may not transfer well across all environments, such as across all patient cohorts. For example, a threshold as set for environment A might not reach a 90.0% sensitivity target when using this threshold also in environments B to D due to differences in the patient cohorts or medical centre environments. In order to still reach a 90.0% sensitivity target in environments B to D, a solution is to perform threshold calibration for each environment and get environment-specific thresholds. This means that it is necessary to repeat the threshold calibration in environments B to D, wherein density plots are also generated in environments B to D, as shown in FIG. 1 by way of example. Thus, density plots 110b, 120b may be generated for environment B, density plots 110c, 120c may be generated for environment C, and density plots 110d, 120d may be generated for environment D. It is noted that the density plots 110b, 110c, and 110d represent the probability density functions of the image scores generated for histological images obtained in the environments B, C, and D, respectively, and labelled with a binary value of "0" (e.g. no MSI present). The density plots 120b, 120c, and 120d represent the probability density functions of the image scores generated for histological images obtained in the environments B, C, and D, respectively, and labelled with a binary value of "1" (e.g. MSI present). Due to the significant differences in the density plots of the environments A to D, the threshold to reach the 90.0% sensitivity target differs for each environment (ranging from 0.15 for environment B to 0.5 for environment A).

Thus, it may be very laborious and tedious to determine adequate thresholds for binary classification. Furthermore, a large number of patients may be needed to be scanned in order to obtain a large number of histological images for each environment for adequate threshold calibration.

Here, a distinction must be made between two different meanings of "calibration". Strictly speaking, calibration in classification problems refers to the transformation of prediction scores into actual class membership probabilities (see also Dawid, A.P.: The Well-Calibrated Bayesian. Journal of the American Statistical Association 77(379), 605-610 (Sep 1982). https://doi.org/10.1080/01621459.1982.10477856), for instance using temperature scaling (see also Guo, C. et al.: On calibration of modern neural networks. In: Precup, D., Teh, Y.W. (eds.) Proceedings of the 34th International Conference on Machine Learning, ICML 2017, Sydney, NSW, Australia, 6-11 August 2017. Proceedings of Machine Learning Research, vol. 70, pp. 1321-1330. PMLR (2017)). While probabilistic model calibration is a way to control model sensitivity, it may be a much stronger requirement as it may aim to control it at any given level. In practice however, it is usually desired to ensure that DL models achieve a fixed sensitivity level prescribed by the application of interest (say, 90% as in FIG. 1). Thus, other works, particularly in the medical field, use "calibration" in its more general meaning, referring to a DL model's capacity to output similar prediction scores at training and inference time. This is often achieved by leveraging a calibration data set from a new deployment site, to adjust the model's operating threshold after training time (see also Saillard, C. et al.: Validation of MSIntuit as an AI-based pre-screening tool for MSI detection from colorectal cancer histology slides. Nature Communications 14(1), 6695 (Nov 2023). https://doi.org/10.1038/s41467-023-42453-6; Roschewitz, M. et al.: Automatic correction of performance drift under acquisition shift in medical image classification. Nature Communications 14(1), 6608 (Oct 2023). https://doi.org/10.1038/s41467-023-42396-y).

### SUMMARY

It may be an object of the invention to provide methods and systems for improved image classification with decreased resources.

According to an aspect, a computer-implemented method for classifying an input image obtained in a first environment is provided. The method comprises tiling the input image into a set of tiles and generating first tile scores for the set of tiles using a machine learning (ML) model. The method further comprises applying a transport map to the generated first tile scores to obtain second tile scores and using the second tile scores in the second ML model to generate an image score for the input image. The image score is compared to a threshold to classify the input image. The transport map indicates a mapping from a first tile score distribution to a second tile score distribution, the first tile score distribution being a distribution of tile scores of a set of images obtained in the first environment and tiled into tiles and the second tile score distribution being a distribution of tile scores of a set of images obtained in a second environment and tiled into tiles. The threshold is with respect to the second environment.

According to another aspect, a system for classifying an input image obtained in a first environment is provided. The system comprises a processing unit configured to tile the input image into a set of tiles and generate first tile scores for the set of tiles using a ML model. The processing unit is further configured to apply a transport map to the generated first tile scores to obtain second tile scores and use the second tile scores in the ML model to generate an image score for the input image. The image score is compared to a threshold to classify the input image. The transport map indicates a mapping from a first tile score distribution to a second tile score distribution, the first tile score distribution being a distribution of tile scores of a set of images obtained in the first environment and tiled into tiles and the second tile score distribution being a distribution of tile scores of a set of images obtained in a second environment and tiled into tiles. The threshold is with respect to the second environment.

According to another aspect, a computer program is provided. The computer program comprises instructions which, when the program is executed by a computer, cause the computer to carry out the above-stated method.

According to another aspect, a computer-readable medium is provided. The computer-readable medium comprises instructions which, when executed by a computer, cause the computer to carry out the above-stated method.

### BRIEF DESCRIPTION OF THE DRAWINGS

FIG. 1 illustrates a state-of-the art threshold calibration by way of example.
FIG. 2 illustrates a flow chart of a method for classifying an input image obtained in a first environment according to an embodiment.
FIG. 3 illustrates a schematic diagram of a setup for image classification according to an embodiment.
FIG. 4 illustrates examples of two density plots for a first environment and a second environment.
FIGs. 5A and 5B illustrate an example of histogram matching and transport map.
FIG. 6 illustrates a schematic diagram of a setup for image classification according to another embodiment.
FIG. 7 illustrates a schematic diagram of a setup for image classification in a first environment according to an example.
FIG. 8 illustrates a schematic diagram for pre-processing whole slide images.
FIG. 9 illustrates a schematic diagram of a system for classifying an input image obtained in a first environment according to an embodiment.
FIG. 10 illustrates ROC curves and sensitivity versus threshold graphs for indicating the presence or absence of MSI in a private collection FTB.
FIG. 11 illustrates, by way of example, a sensitivity achieved by several methods on various tasks and validation cohorts.
FIG. 12 illustrates, by way of example, a sensitivity by several methods using 5 positive samples for calibration.

### DETAILED DESCRIPTION

Some of the embodiments contemplated herein will now be described more fully with reference to the accompanying drawings. Other embodiments, however, are contained within the scope of the subject matter disclosed herein, the disclosed subject matter should not be construed as limited to only the embodiments set forth herein; rather, these embodiments are provided by way of example to convey the scope of the subject matter to those skilled in the art.

Generally, all terms used herein are to be interpreted according to their ordinary meaning in the relevant technical field, unless a different meaning is clearly given and/or is implied from the context in which it is used. All references to a/an/the element, apparatus, component, means, step, etc. are to be interpreted openly as referring to at least one instance of the element, apparatus, component, means, step, etc., unless explicitly stated otherwise. The steps of any methods disclosed herein do not have to be performed in the exact order disclosed, unless a step is explicitly described as following or preceding another step and/or where a step must necessarily follow or precede another step due to some dependency. Any feature of any of the embodiments disclosed herein may be applied to any other embodiment, wherever appropriate. Likewise, any advantage of any of the embodiments may apply to any other embodiments, and vice versa. Other objectives, features, and advantages of the enclosed embodiments will be apparent from the following description.

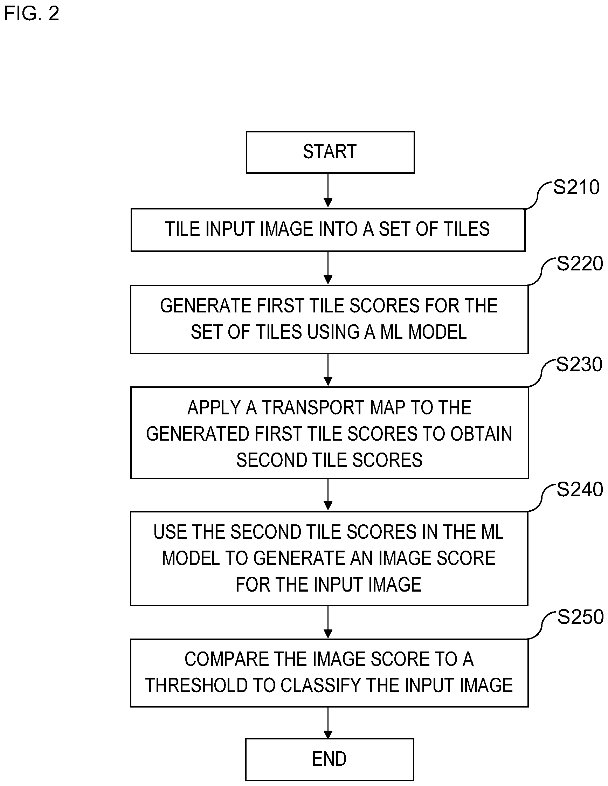

FIG. 2 illustrates a flow chart of a method for classifying an input image obtained in a first environment according to an embodiment. The first environment may be a first medical centre environment, such as a first hospital environment, or may be a first patient cohort, also called validation cohort. The first hospital environment may be a first hospital. Thus, the input image obtained in the first environment may be generated or obtained by machines, measurement equipments, etc., in the first medical centre environment or for a first patient cohort.

The input image may be a histological image. For example, the histological image is a digitalized image, a whole slide image (WSI), a digitalized WSI, or the like, of a histological section. The histological image may be a microscopic image of tissues that may be used in the analysis of diseases, such as cancer diagnosis. For example, the histological image shows two-dimensional (2D) slices from a three-dimensional (3D) piece of tissue. The tissues may have been stained to highlight different cellular components and structures. For example, the histological section may be stained with a dye, such as Haematoxylin and Eosin (H&E), Haematoxylin Phloxine saffron (HPS), Hematoxylin Eosin Saffron (HES), or the like.

The analysis of histological images, including histological image classification, allows pathologists or diagnosis tools to diagnose diseases, understand cellular interactions, and conduct detailed anatomical studies. The accuracy of histological image analysis is therefore important since it directly impacts clinical decisions and the understanding of complex biological processes.

As illustrated in FIG. 2, the method for classifying the input image may comprise tiling the input image (S210) into a set of tiles and generating (S220) first tile scores for the set of tiles using a machine learning (ML) model. Thus, the ML model may be a model able to generate a first tile score for each tile in the set of tiles, such that each tile of the set of tiles is assigned to a first tile score. The tile scores can take on any values and is not restricted to a positive value or a value between 0 and 1. The tile scores can also take on a negative value or values outside the range between 0 and 1.

Generating first tile scores for the set of tiles using the ML model may include the generation of tile characteristics for each tile of the set of tiles, wherein the tile characteristics may be input in the ML model to obtain the tile scores. The tile characteristics may be features vectors or other local descriptors generated for the image data mentioned above.

For instance, feature vectors including a plurality of features are extracted for the set of tiles, wherein the features of the feature vectors represent local descriptors of each tile in the set of tiles. Thus, a plurality of feature vectors may be generated for the set of tiles, each tile being assigned to a respective feature vector. This means that a respective feature vector can be extracted for each tile from the set of tiles. The first tile scores may be generated from the feature vectors using the ML model. Therefore, for each feature vector, a respective tile score may be generated.

This means that the tiles in the set of tiles may be encoded to vectors to have a simple feature presentation. The feature vectors may be a collection of numbers, typically a few hundreds to a few thousands, encoding information about the tiles. The extraction may be done using, for example, a pre-trained foundation model which is able to extract the feature vectors for each tile.

There may be no immediate and simple correspondence between the numbers included in the feature vectors and physical characteristics of the tiles. Instead, the pre-trained foundation model may "compress" the tiles in a most efficient way. For example, a given pre-trained foundation model may reduce each tile to a feature vector, i.e. to an abstract descriptor, while trying to encode the relevant information from each tile.

The pre-trained foundation model may be a model trained with Momentum Contrast (MoCo), which is a self-supervised learning algorithm with a contrastive loss, or may be a Phikon model, which is a self-supervised learning model for histopathology, such as histological images, trained with iBOT. iBOT is a self-supervised learning algorithm with a Masked Image Modelling (MIM) loss. However, this is not limiting, and any other pre-trained foundation model for feature extraction may be used. As indicated above, the plurality of feature vectors may represent or encode local descriptors of each tile, such as local characteristics of the tiles. The local characteristics of the tiles may be features of the tiles, such as cell types present in the tiles, as opposed to global descriptors of the tiles, such as total number of cells present in the tiles, average colour of the tiles, etc.

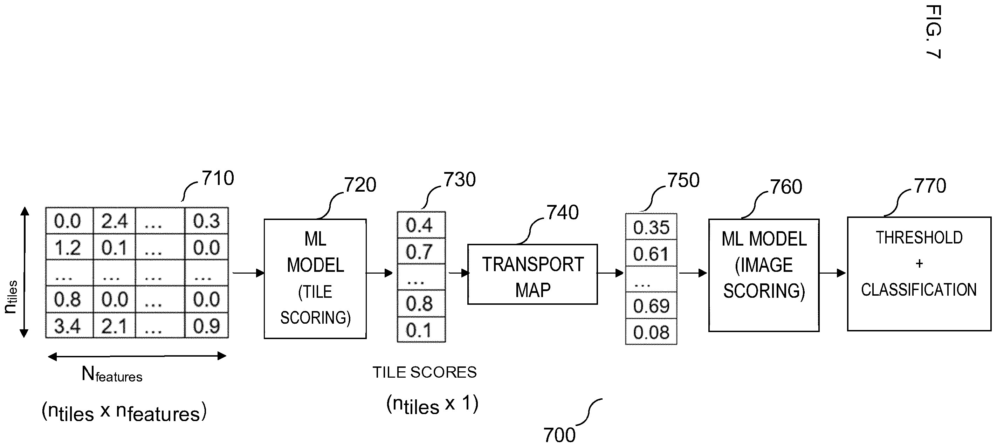

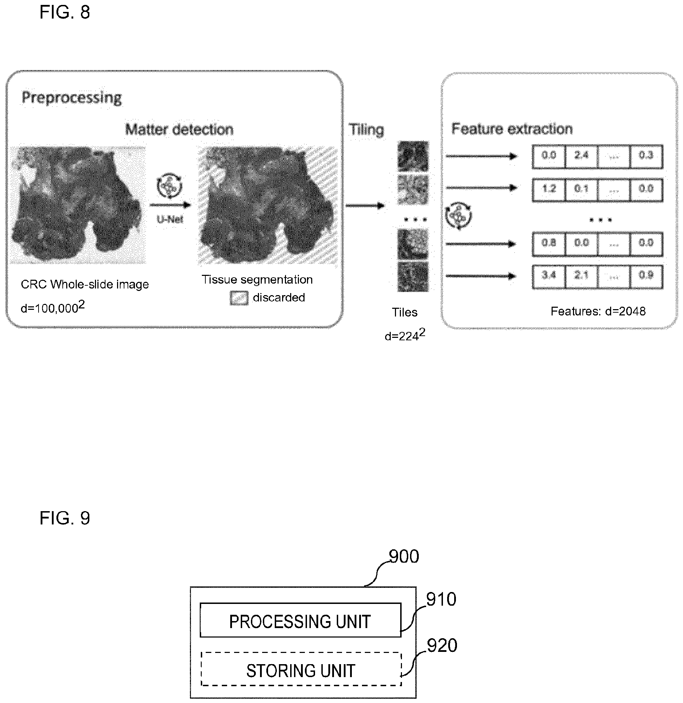

The ML model may generate the tile scores for the set of tiles using the respective feature vectors. By extracting feature vectors and generating the tile scores based on these feature vectors, the data processed by the ML model can be reduced, leading to a reduced workload of the ML model compared to a case where the ML model processes the entire tiles to generate the tile scores (this is also further described with respect to FIGs. 7 and 8 below).

Returning to FIG. 2, after the first tile scores have been generated, the method may further comprise applying (S230) a transport map to the generated first tile scores to obtain second tile scores. Some or all of the second tile scores may differ from the first tile scores. For example, when applying the transport map, a function, such as a one-dimensional (1D) function, may be used to transform, i.e. modify, the first tile scores one-by-one to the second tile scores.

The second tile scores may be used (S240) in the ML model to generate an image score for the input image. This means that the ML model generates an image score for the input image based on the second tile scores. Thus, a prediction at image-level is provided by considering the tile scores for a set of tiles obtained from the input image. In other words, the image score for the input image is predicted, i.e. generated, from the tile scores assigned to the tiles of the input image. The image score may be a value between 0 and 1.

According to an example, the ML model is a trained ML model being trained on input-output data pairs. For example, the input-output data pairs are a training data set comprising image data as input data and corresponding image scores as output data. The image scores may be understood as a descriptor or label for each image data, differing depending on characteristics in the image data. The image data may be histological images, such as WSI, or parts of the histological images, wherein background sections in the histological images have been removed due to segmentation, for example. Thus, by training the ML model based on such a training data set, it is possible to input unknown tiles, obtained from an unknown input image, in the trained ML model, generate tile scores for the tiles, and obtain a corresponding image score for the unknown input image comprising the tiles. For example, the ML model is a neural network, multi-layer perceptron (MLP), or linear model. If the ML model is used on feature vectors, instead of tiles, the ML model may be trained on feature vectors as input data and respective image scores as output data. The ML model may be trained end-to-end by cross entropy loss minimization, wherein a loss may be computed by comparing the output of the ML model to the output data in the training data set and gradient descent may be applied to this loss. The training data set may be generated by a pathologist, for example, assigning labels, such as image scores, to the image data. This is, however, not limiting. For instance, molecular testing can be used to assign image data to its label (as for MSI prediction, for example).

As mentioned above, the ML model may be a neural network, multi-layer perceptron (MLP), or linear model. The ML model may comprise several blocks, sub-models, or layers, such as an input layer, several hidden layers, and an output layer in a neural network, wherein the tile scores generated may be internal representations learned by the ML model as a byproduct of the training. For example, the tile scores are generated after a block, sub-model, or hidden layer in the neural network, wherein the image score is output by the output layer of the ML model. Thus, the architecture of the ML model can be divided internally into two specific blocks or sub-models, wherein one block or sub-model is used to compute the tile scores as intermediate result of the ML model and another block or sub-model is used to compute the image scores as final result of the ML model. The blocks and sub-models may be trained end-to-end using the same training data set as explained above.

According to another example, the sub-models can be trained separately. For example, the ML model comprises a first sub-model and a second sub-model. The first sub-model and the second sub-model may be neural networks, multi-layer perceptrons (MLP), or linear models, such as logistic regression models. The first sub-model may be a trained ML model being trained on first input-output data pairs, wherein the first input-output data pairs may comprise the image data (as described above) as input data and corresponding tile scores as output data. Instead of the image data, also feature vectors may be used as input data.

The second sub-model may be trained differently from the first sub-model. For example, the second sub-model is a trained ML being trained on second input-output data pairs as training data set. The second input-output data pairs may comprise tile scores as input data and corresponding image scores as output data. As indicated above, the image scores may be understood as a descriptor for each input image, differing depending on image characteristics, wherein each input image may be assigned to a respective image score.

Thus, regardless of whether the ML model is trained end-to-end based on a training data set or first and second sub-models of the ML model are trained separately based on a plurality of training data sets, it is possible to input tiles in the ML model to obtain the tile scores as intermediate result, wherein the tile scores are used in the ML model to obtain a corresponding image score for the input image as final result.

As further illustrated by FIG. 2, the image score may be compared (S250) to a threshold to classify the input image. Thus, a multiple instance learning (MIL) architecture is provided, wherein a binary output can be generated using the threshold.

However, in the first environment, no threshold calibration as, for example, described with respect to FIG. 1 is performed. There may only be a threshold with respect to a second environment. The second environment may be different from the first environment, e.g. a second medical centre environment, such as a second hospital environment, or a second patient cohort, also called train cohort, wherein machines, measurement equipment, patient demographics, etc. with respect to the second medical centre environment may differ from the first medical centre environment. The second hospital environment may be a second hospital. As shown with respect to FIG. 1, the threshold can usually not be transferred to other environments due to the differences in the environments and may need to be adjusted to reach pre-specified sensitivity or specificity targets, for example.

In order to classify the input image by comparing the image score of an input image obtained in the first environment to a threshold with respect to the second environment, the above-described transport map has been applied. In other words, taking the threshold with respect to the second environment also for the first environment is possible because of the step of applying the transport map. The transport map may indicate a mapping, i.e. a transport, from a first tile score distribution to a second tile score distribution, wherein the first tile score distribution may be a distribution of tile scores of a set of images obtained in the first environment and tiled into tiles. The second tile score distribution may be a distribution of tile scores of a set of images obtained in the second environment and tiled into tiles. According to an example, the transport map is a solution of an optimal transport problem, wherein the first tile score distribution may be transported in an optimal way to minimize a cost function, see also https://math.univ-lyon1.fr/~santambrogio/OTAM-cvgmt.pdf, especially chapter 2 on the specificities of one dimensional (1D) use cases. For instance, the transport map is a Monge map. Further details regarding the transport map are given below.

Thus, the tedious and laborious step of threshold calibration for the first environment can be skipped by using a transport map on the tile scores, allowing the usage of the threshold obtained in the second environment also in the first environment. This means that the threshold calibration does not need to be repeated for the first environment when a threshold calibration has already been performed in a different environment, here the second environment. By introducing the transport map, distribution shifts in predictions can be handled in an efficient way when deploying image classification in a new environment. This is possible, because it is assumed that the area under receiver operating characteristic (ROC) curves (ROC-AUC) with respect to the distributions can transfer between the first environment and the second environment.

Such an image classification method as described above can be performed for any architecture which produces an internal 1D representation of tiles, here the tile scores, wherein the tile score distributions between a first environment and a second environment can be matched using a transport map. This allows the subsequent layers of the architecture to act in a pseudo-in-domain fashion, meaning that after the transport mapping step, the tile scores are seen by the subsequent steps performed in the architecture as being from the second environment. The image classification method can be included in a diagnostic tool, such as the diagnostic tool MSIntuit^{®}CRC (a CE-marked AI diagnostic that provides a pre-screen approach with digital pathology), in order to provide accurate diagnosis for patients.

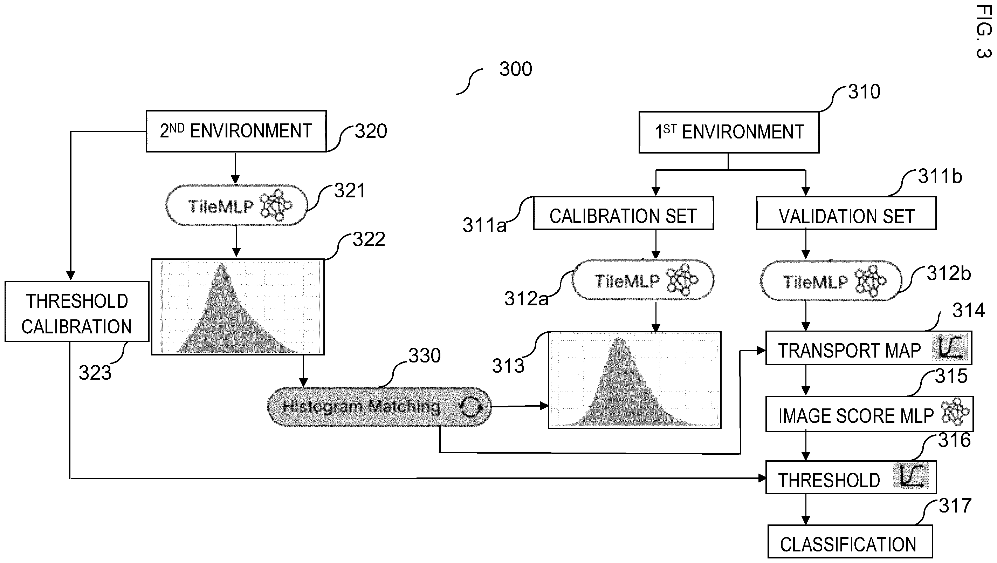

FIG. 3 illustrates a schematic diagram of a setup 300 for image classification according to an embodiment. The method performed with respect to FIG. 3 may also be called "unsupervised method". In FIG. 3, a first environment 310 and a second environment 320 are illustrated. As indicated above, the first and second environments 310 and 320 may be different medical centre environments or different patient cohorts.

In the second environment 320, a threshold calibration 323 may be performed to generate a threshold. For example, the threshold is determined or selected in the second environment 320 based on a pre-specified sensitivity target or a pre-specified specificity target. Thus, the threshold may be determined or selected in such a way that a pre-specified sensitivity target or a pre-specified specificity target may be reached.

According to an example, the threshold may be determined similar to the method described with respect to FIG. 1. For example, the threshold is determined by generating at least one labelled image score distribution, the at least one labelled image score distribution being a distribution of image scores of a set of labelled images obtained in the second environment 320. The threshold may be determined using the at least one labelled image score distribution, wherein the threshold is set in the second environment 320 so as to reach the pre-specified sensitivity target or the pre-specified specificity target. For instance, for a binary classification, there may be two labelled image score distributions, one image score distribution for images labelled with "1", for example, and another image score distribution for images labelled with "0", for example. As indicated above, the images labelled with "0" may be images in which no specific biomarker, no specific gene mutation, no tumour cells, etc. are present. The images labelled with "1" may be images in which specific biomarker, specific gene mutation, tumour cells, etc. are present, for example. For obtaining the image scores, these labelled images, obtained in the second environment, may be tiled into tiles, wherein tile scores and image scores may be generated for these tiles as described above, for example by using the ML model. For these image scores, two separate labelled image score distributions may be generated, one labelled image score distribution being a density plot to indicate the probability density function of the image scores generated for the images obtained in the second environment and labelled with a binary value of "0", and the other labelled image score distribution being a density plot to indicate the probability density function of the image scores generated for the images obtained in the second environment and labelled with a binary value of "1". The threshold may be determined by considering both labelled image score distributions, or one of the labelled image score distributions. Once a threshold is determined, an image having an image score below or equal to the threshold may be classified as having a label "0", for example, and an image having an image score above the threshold may be classified as having a label "1", for example. However, this is not limiting, and the images may be classified differently with respect to the threshold.

As mentioned above, the transport map may indicate a mapping from a first tile score distribution to a second tile score distribution. This is further explained by way of example with respect to FIG. 3. The first tile score distribution may be a density plot 313 computed from tile scores of a set of images obtained in the first environment 310. For example, the set of images obtained in the first environment 310 is a calibration set 311a which is tiled into tiles. For each tile, a tile score is generated using a ML model 312a, also called "TileMLP" in FIG. 3. The ML model 312a may be the ML model as described above to generate the tile scores. The x-axis of the density plot 313 may represent the tile scores, whereas the y-axis may represent the density of the tile scores.

The same is done in the second environment 320. The second tile score distribution may be a density plot 322 computed from tile scores of a set of images obtained in the second environment 320. The set of images may be the same as the images used for the threshold calibration. The set of images may be tiled into tiles, wherein tile scores may be generated for each tile using a ML model 321, also called "TileMLP" in FIG. 3. The ML model 321 may be the same model as ML model 312a. For example, the ML model 321 is the ML model described above to generate the tile scores. The x-axis of the density plot 322 may represent the tile scores, whereas the y-axis may represent the density of the tile scores.

It is noted that the embodiment is not limited to using density plots. It is also possible to use histogram plots or histogram density plots instead. In such a case, the curves 322, 313 may be histogram plots or histogram density plots.

It is further noted that a number of images included in the set of images 311a obtained in the first environment 310 and used for the first tile score distribution 313 may be smaller than a number of images included in the set of images obtained in the second environment 320 and used for the second tile score distribution 322. For instance, the calibration set 311a may comprise merely 30 or even less than 30 images which are enough to generate the first tile score distribution 313 after tiling the images into tiles. Thus, less resources are needed in the first environment 310 compared to a case where threshold calibration as done in the second environment 320 (see the threshold calibration 323) is performed, because usually several dozens, hundreds or even thousands of images are needed for threshold calibration. This is especially advantageous in cases when a number of available images having a positive label, i.e. images showing the presence of a biomarker, gene mutation, tumour cells, etc., is low in a threshold calibration phase. Thus, the deployment of a diagnostic model with reduced resources is allowed.

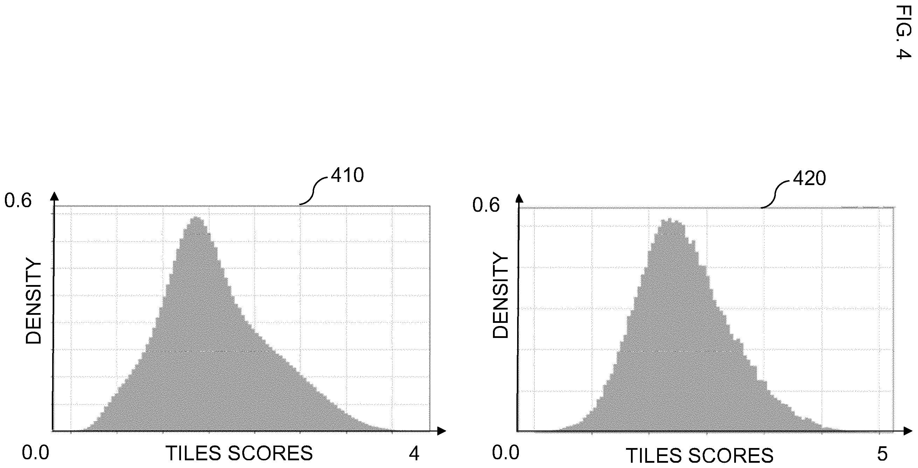

A histogram matching 330, also called a density plot matching, may be performed using these two density plots 313 and 322. Examples of two density plots 410 and 420 are also illustrated in FIG. 4 by way of example. The density plot 410 may be the density plot 322 obtained in the second environment 320 and the density plot 420 may be the density plot 313 obtained in the first environment 310. The x-axis of the density plots 410 and 420 may represent the tile scores, which can be any value, and the y-axis of the density plots 410 and 420 may represent the density of the tile scores, the density being values between 0 and 1. As can be seen from FIG. 4, the general form of the density plots 410 and 420 differ slightly. Again, the histogram matching 330 is not limited to matching density plots. It is also possible to use histogram plots or histogram density plots instead. In such a case, the curves 410, 420 may be histogram plots or histogram density plots.

In order to be able to use the threshold determined in the second environment also in the first environment, the density plot 420 may be mapped, i.e. transported, to the density plot 410. This mapping problem may be formulated as an optimal transport problem, which has a well-known closed-form solution through quantile matching in 1D. For example, it is desired to find a Monge map between the 1D density plots 410 and 420. The obtained transport map may exist, may be unique, and may be monotonous. It is again referred to https://math.univ-lyon1.fr/~santambrogio/OTAM-cvgmt.pdf as an example of how a solution to an optimal transport problem can be found (see again especially chapter 2 on the specificities of one dimensional (1D) use cases).

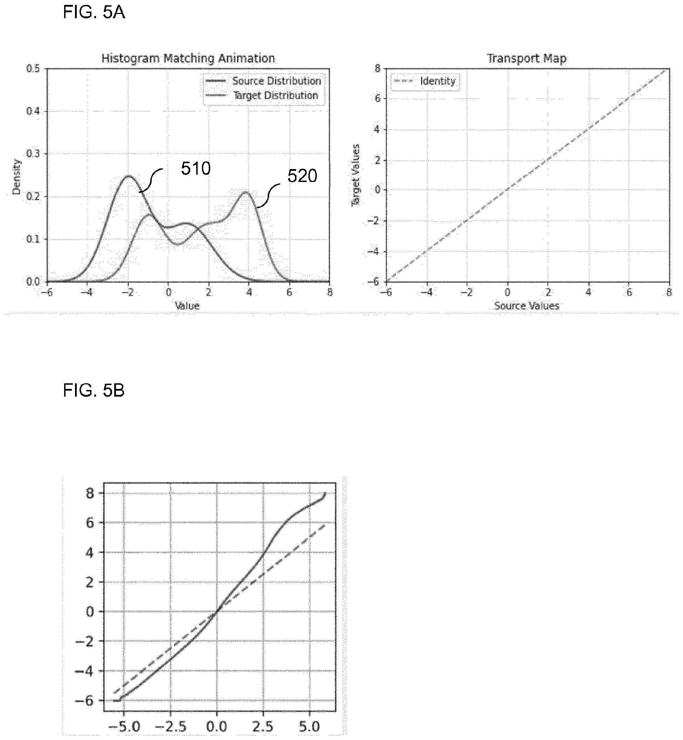

The histogram matching is further illustrated in FIGs. 5A and 5B. FIG. 5A illustrates a histogram matching on the left side and an example of a transport map on the right side, whereas FIG. 5B shows another example of a transport map. FIG. 5A shows a source distribution 510 and a target distribution 520. The source distribution 510 may be the density plot 313 or 420 which needs to be matched to the target distribution 520, such as the density plot 322 or 410. For the optimal transport problem, a 1D function as illustrated in FIG. 5B (x-axis: source values; y-axis: target values) with the solid line may be found as the transport map mapping the source distribution 510 (the source values represented by the x-axis) to the target destination 520 (the target values represented by the y-axis). An identity function is illustrated in FIG. 5A, right side, and in FIG. 5B with the dashed lines. It is indicated that the 1D function found as the transport map (solid line in FIG. 5B) differs from the identity function (dashed line in FIG. 5A, right side, or FIG. 5B). The identity function illustrates a case where no transport map is applied. The differences between the solid line and the dashed line show that the tile scores are effectively moved, i.e. transported, by applying the transport map.

The output of the histogram matching 330 may be a transport map 314 which may be used on the first tile scores described above. The transport map 314 may be a 1D function as illustrated in FIG. 5B (solid line) by way of example.

According to an example, the image classification may be performed on a validation set 311b in the first environment 310. The validation set 311b may comprise an input image for which the image classification should be performed. As described above, the input image may be tiled into tiles, wherein first tile scores may be generated for the tiles using a ML model 312b, also called "TileMLP" in FIG. 3. The ML model 312b may be the same model as the models 312a and 321, and may be the ML model described above to generate the tile scores.

After the first tile scores have been generated, the transport map 314 obtained from the histogram matching 330 may be applied to the first tile scores to obtain the above-stated second tile scores. As mentioned above, the transport map 314 may be a function, such as a 1D function, which may be used to transform, i.e. modify, the first tile scores one-by-one to the second tile scores. Thus, the subsequent steps in FIG. 3 may act in a pseudo-in-domain fashion.

These second tile scores may be input to a ML model 315, also called "IMAGE SCORE MLP" in FIG. 3. The ML model 315 may be the ML model described above to generate image scores. For example, the ML models 312b and 315 may refer to different blocks, sub-models, or layers in the ML model. The output of the ML model 315 may be an image score for the input image of the validation set 311b. This image score may be compared to a threshold 316, obtained by the threshold calibration 323 in the second environment, to perform classification 317 of the input image. Thus, a continuous score, for instance an image score between 0 and 1, may be produced that is then "thresholded", i.e. compared to a threshold, to produce a final classification decision.

According to an embodiment (not only limited to the setup shown in FIG. 3), the image score may be compared to the threshold to obtain a binary classification of the input image. Binary classification may mean that there are two image classes, wherein the input image is assigned to one of the two image class. For instance, a binary output is output after comparing the image score to the threshold to obtain the binary classification, wherein a patient, from whom the input image is taken or acquired, may be diagnosed based on the binary output. The binary output may be represented by a single bit, which can take on only one of two possible states. This means that the binary output can be one of these two values. For example, the binary output is "0" or "1". Thus, assigning the input image to one of the two image classes may comprise assigning the input image to one of two labels, such as "0" and "1", for example. However, this is not limiting, and any other label can be chosen.

According to an example, the binary classification indicates presence or absence of a biomarker, indicates presence or absence of a gene mutation, indicates presence or absence of tumour cells, indicates a patient being at high risk or at low risk of an adverse clinical outcome, indicates a patient having a low survival rate or a high survival rate, or the like. The adverse clinical outcome may be a specific disease. The low or high survival rate may be with respect to a specific disease, such as cancer.

For example, the input image is classified as having the binary output "0" when its respective image score is below or equal to the threshold, and the input image is classified as having the binary output "1" when its respective image score is greater than the threshold. The binary output of "0" may indicate that the input image does not include a biomarker, a gene mutation, tumour cells, or the like, or may indicate that a patient is at low risk of an adverse clinical outcome or has a high survival rate. The binary output of "1" may indicate that the input image includes a biomarker, a gene mutation, tumour cells, or the like, or may indicate that a patient is at high risk of an adverse clinical outcome or has a low survival rate. However, this is not limiting, and any other binary output may be chosen.

Thus, based on the binary output, a diagnosis for a patient, from whom the input image has been taken, can be provided. For example, a diagnosis report is generated for the patient based on the binary output. The diagnosis report may include information regarding the biomarkers, gene mutations, or tumour cells found, may include information regarding the risk of an adverse clinical outcome, or may include information regarding the survival rate. This image classification and diagnosis can be performed after applying the threshold, see box 317 in FIG. 3.

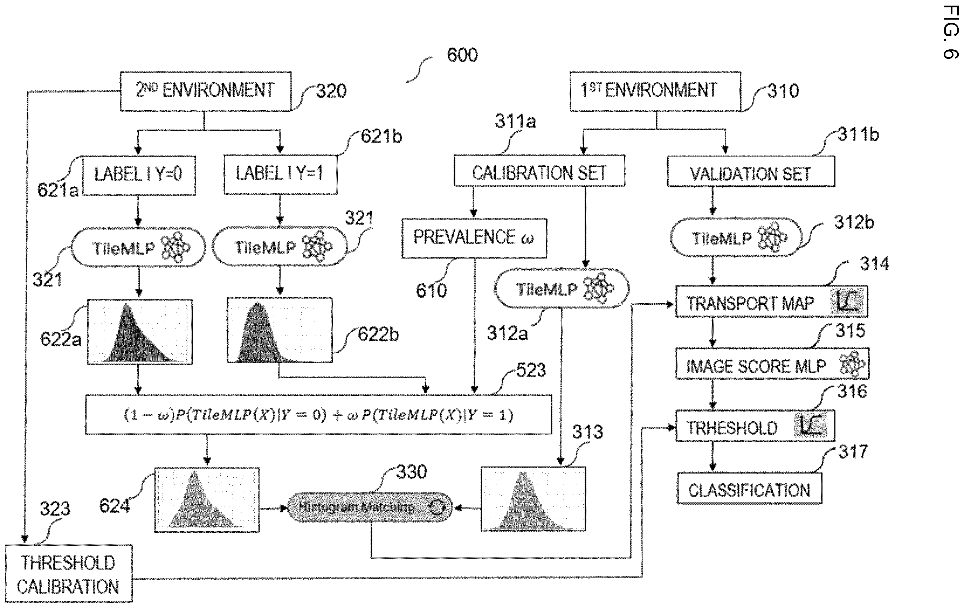

As shown in FIG. 3, the tile score distributions 313 and 322 are matched without requiring any additional information, such as labels of the images. This may also be called an unsupervised setting or unsupervised method. In order to further improve the image classification, a supervised setting coupled with importance sampling, also called supervised method or tile score matching with importance sampling (TSM-IS), may be applied which is shown by way of example with respect to FIG. 6. The importance sampling refers to the fact that a constraint may be added to match a prevalence value from the first environment, as further discussed below.

FIG. 6 illustrates a schematic diagram of a setup 600 for image classification according to another embodiment. In the setup 600, there are again a first environment 310 and a second environment 320 given, which are similar to the first environment 310 and second environment 320 illustrated in FIG. 3. The reference signs being the same in FIG. 3 and FIG. 6 refer to the same units. Thus, it is noted that the threshold calibration 323, the MLP models 321, the histogram matching 330, the ML model 312a, ML model 312b, transport map 314, ML model 315, threshold 316, and classification 317 in FIG. 6 are equal to the threshold calibration 323, the ML model 321, the histogram matching 330, the ML model 312a, ML model 312b, transport map 314, ML model 315, threshold 316, and classification 317 in FIG. 3. For conciseness reasons, it is therefore referred to FIG. 3 for a detailed description.

The difference between FIG. 3 and FIG. 6 is that, in FIG. 3, the second tile score distribution is obtained based on a set of unlabelled images obtained in the second environment and tiled into tiles, whereas, in FIG. 6, the second tile score distribution is obtained based on a set of labelled images obtained in the second environment and tiled into tiles. Thus, a setting such as in FIG. 6 may be considered when histological images with labels exist. The setting of FIG. 6 is further described in detail below.

As shown in FIG. 6, the set of labelled images may comprise a first group of images 621a labelled with a first label, here labelled with a label "0" (see also "LABEL|Y=0" in FIG. 6), and a second group of images 621b labelled with a second label different from the first label, here labelled with a label "1" (see also "LABEL|Y=1" in FIG. 6). The labels may have been provided by a pathologist or previous classifications done on the images, for example. However, this is not limiting, and the labels may be provided differently, such as by performing molecular testing, for example. The first group of images 621a may be used to generate a first labelled tile score distribution 622a, whereas the second group of images 621b may be used to generate a second labelled tile score distribution 622b. For example, the first labelled tile score distribution 622a is a distribution of tile scores of the first group of images 621a tiled into tiles. For example, the second labelled tile score distribution 622b is a distribution of tile scores of the second group of images 621b tiled into tiles. Thus, the first and second labelled tile score distributions 622a and 622b may be obtained by using the labelled images 621a and 622b obtained in the second environment 320, wherein the labelled images 621a and 622b may be tiled into tiles and tile scores may be generated for the tiles using the ML model 321. The ML model 321 may be the ML model described above. The first and second labelled tile score distributions 622a and 622b may be generated from the generated tile scores. Hence, there are two labelled tile score distributions, the first labelled tile score distribution 622a for the images labelled with "0" and the second labelled tile score distribution 622b for the images labelled with "1".

As further shown in FIG. 6, the first labelled tile score distribution 622a and the second labelled tile score distribution 622b may be combined using a prevalence value ω from the first environment to obtain the second tile score distribution 624. The prevalence value ω may be reported as a percentage (e.g. 5%, or 5 people out of 100) or as a number of cases per 10,000 or 100,000 people. The way the prevalence value ω is reported may depend on how common a characteristic, such as a biomarker or gene mutation, is in a population or patient cohort. Thus, the prevalence may be a proportion of a particular population found to be affected by a medical condition at a specific time. This means that the prevalence value ω may represent a proportion of patients for which the histological images may be labelled with "1", i.e. positive patients, and may be a value between 0 and 1.

The first labelled tile score distribution 622a and the second labelled tile score distribution 622b may be combined using the following equation: $\left(1-ω\right) P \left(TileMLP\left.\left(X\right)\right|Y=0\right) + ω P \left(TileMLP\left.\left(X\right)\right|Y=1\right)$ wherein ω is the prevalence value obtained from the first environment 310, ***P(TileMLP(X)IY =* 0)** represents the first labelled tile score distribution 622a, and ***P(TileMLP(X)IY =* 1)** represents the second labelled tile score distribution 622b.

Once the second tile score distribution 624 is obtained, histogram matching 330 may be performed between the second tile score distribution 624 and the first tile score distribution 313 obtained as described with respect to FIG. 3. The subsequent steps illustrated with 312b, 314, 315, 316, and 317 are as described with respect to FIG. 3.

As shown above in FIG. 6, in the supervised setting when coupled with importance sampling, it is possible to handle the images of the first and second environments 310 and 320 with varying prevalence, enforcing an additional prevalence constraint on the first and second environments 310 and 320. Such a supervised setting may be especially advantageous when only a small number of positive histological images is available, for example when working on diseases where the positive histological images, i.e. images showing the disease, have a low prevalence, and are therefore costly to obtain. By this supervised setting, a distribution with a same prevalence as in the first environment 310 is mimicked in the second environment 320, wherein the distribution with the same prevalence is used for histogram matching 330. Thus, a more accurate transport map 314 can be generated, leading to a more accurate transformation to the second tile scores and to a more accurate image classification.

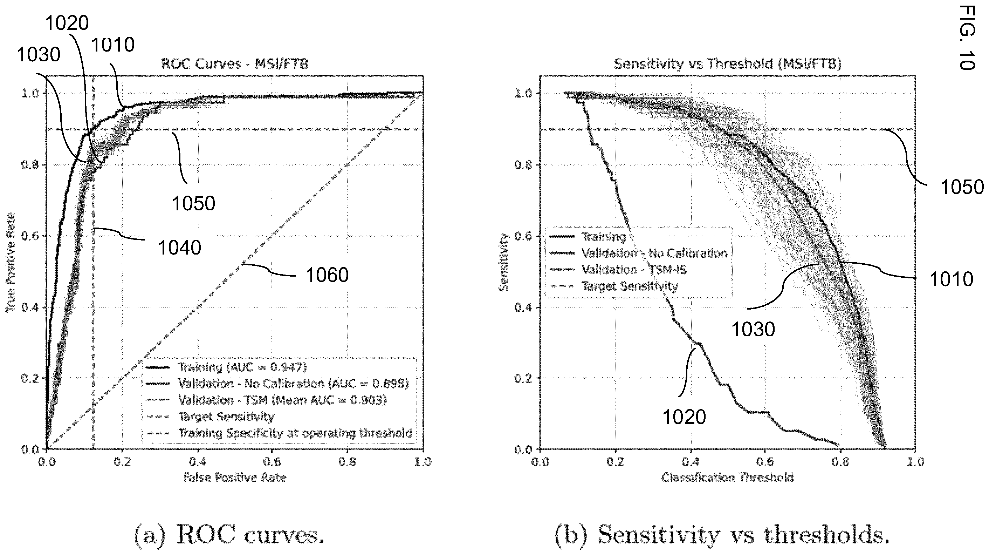

To sum it up, the settings 300 and 600, illustrated in FIGs. 3 and 6 by way of example, can efficiently be leveraged to calibrate a threshold and reach pre-specified sensitivity or specificity targets both the first and second environments 310 and 320, also in situations where there is access to only a few positive histological images during the threshold calibration phase. This is also further illustrated in FIGs. 10 to 12 below.

FIG. 7 illustrates a schematic diagram of a setup 700 for image classification in a first environment according to an example. An input image obtained in the first environment may be tiled into a set of tiles. In FIG. 7, feature vectors 710 are shown by way of example for each tile, wherein there are nₜᵢₗₑₛ feature vectors, nₜᵢₗₑₛ being the number of tiles in the set of tiles for which the feature vectors have been generated. Each feature vector has a length of N_{features}, N_{features} being the number of features included in each feature vector. Thus, the feature vectors may also be illustrated as a matrix of nₜᵢₗₑₛ × N_{features}.

In order to generate the first tile scores 730 for each tile, the feature vectors may be input in the ML model (described in detail above) for generating tile scores (see box 720 in FIG. 7), wherein the output is the first tile scores 730. Thus, the matrix of nₜᵢₗₑₛ × N_{features} may be reduced to nₜᵢₗₑₛ × 1, meaning that each tile is assigned to one first tile score. It is noted that generating feature vectors for obtaining the first tile scores is an optional step. It is also possible to input the tiles in the ML model to obtain the first tile scores 730.

As explained above, a transport map 740 may be applied to the first tile scores 730 to obtain the second tile scores 750. These second tile scores 750 may be input in the ML model (described in detail above) for obtaining an image score for the input image (see box 760 in FIG. 7). The image score is compared to a threshold with respect to a second environment in order to classify the input image, see box 770 in FIG. 7.

It is noted that the above-described method for image classification has several advantages over state-of-the-art methods. For example, Deep Coral (as mentioned in https://arxiv.org/abs/1607.01719), as an example of a state-of-the-art method, is an example of a strong baseline in domain generalization problems. Deep Coral is designed to align the feature vectors of the tiles after the feature vector extraction step, using a feature extractor or a foundation model mentioned above, between a distribution of a first environment and a distribution of a second environment post-training. However, Deep Coral degrades the performance of a model for image classification as it operates in high dimensional domains. Specifically, Deep Coral operates directly in the feature space (see reference sign 710 in FIG. 7) which is in high dimension (feature size in a range of approximately 1000), whereas the method for image classification, as described herein e.g. with respect to FIGs. 2 to 7, operates on the tile scores, which is later in a dimension of 1 (see reference sign 730 in FIG. 7). Furthermore, there is no guarantee that Deep Coral will preserve the performance (i.e., the AUC) of an image classification method. These disadvantages by state-of-the-art methods can be overcome by implementing a method for image classification as described herein.

FIG. 8 illustrates a schematic diagram for pre-processing an input image. As mentioned above and as shown in FIG. 8, the input image may be a histological image, such as a whole slide image. The whole slide image in FIG. 8 may be a whole slide image showing colorectal cancer (CRC), see also the expression "CRC Whole-slide image" in FIG. 8. The parameter d in FIG. 8 refers to a size of an image in pixels or to a length of a vector. For example, the size of the whole slide image is d=100.000². In order to reduce the workload on the classification process, the whole slide image may be preprocessed before being tiled into tiles. For example, a ML model, such as a U-Net model, or any segmentation method may be used to perform tissue segmentation. The tissue segmentation may be performed to indicate background sections in the whole slide images and discard the background sections, since these sections do not include any information regarding the tissue illustrated in the whole slide image. Only the sections not being background may then be tiled into tiles, wherein feature vectors may be extracted for each tile. The tiles may be small patches, preferably non-overlapping, of a specific size of pixels. For example, the tiles have a size of d=224² (224x224 pixels), whereas the feature vectors may have a length of d=2048, i.e. 2048 features may be included in each feature vector. Thus, by extracting feature vectors, the workload on the ML models to generate tile scores and image scores can be reduced. It is noted that the sizes of the tiles and the feature vectors are not limited to d=224² and d=2048, and any other size can be selected. For example, the dimensions of the feature vectors can depend based on a feature extractor used for extracting the feature vectors.

In order to further reduce the workload on the classification process, it is also possible to reduce the number of tile scores considered for the image score. There are two options how the number of tile scores can be reduced. It is possible to either select a subset from the first tile scores or select a subset from the second tile scores. It is noted that it is not necessary to extract feature vectors beforehand, and the subset can be selected from tile scores generated in any possible way.

For the first option, a subset from the second tile scores may be selected. When comparing this to the setup 700 shown in FIG. 7, the subset may be selected from the second tile scores 750, wherein the selected subset may be used in the ML model to generate the image score. For example, the subset comprises a predetermined number of the highest second tile scores and a predetermined number of the lowest second tile scores. For instance, the second tile scores may be sorted and the 5 highest second tile scores and the 5 lowest second tile scores may be selected for determining the image score. The number "5" is not limiting and any other number can be selected.

For the second option, a subset from the first tile scores may be selected. When comparing this to the setup 700 shown in FIG. 7, the subset may be selected from the first tile scores 730, wherein the transport map may be applied only to the selected subset to obtain the second tile scores. Thus, the second tile scores are also reduced in number, wherein the image score may be obtained from the reduced number of second tile scores. For example, the subset comprises a predetermined number of the highest first tile scores and a predetermined number of the lowest first tile scores. For instance, the first tile scores may be sorted and the 5 highest first tile scores and the 5 lowest first tile scores may be selected for determining the image score. The number "5" is not limiting and any other number can be selected.

Thus, by selecting a subset from the first or second tile scores, the subset having a reduced size compared to the original first or second tile scores, the image score may be generated from this reduced subset of tile scores, reducing the workload on the ML models. Furthermore, when only the highest and lowest tile scores are considered, it is ensured that the extreme tile scores are considered for generating the image score, the extreme tile scores ensuring accurate image score generation.

In machine learning, a common way to improve the performance of a system is to design several models from the same data (instead of one) and use these several models as an "ensemble of models". It is noted that the above-described image classification method can be executed by the ensemble of models operating in parallel, wherein each model may classify the same input image in parallel and may match the tile score distributions in each model. This means that the tile score distribution matching may be performed by each model separately. Thus, a plurality of image classification results may be obtained from the ensemble of models. The final classification result of the input image can then be determined from the plurality of image classification results, for instance by taking the majority, the mean, etc. of the plurality of image classification results output by the ensemble of models. Considering several models in parallel may often result in increased performance and accuracy.

Above, a novel post-training approach has been introduced to control the sensitivity or specificity of image classification models, for example, that leverages optimal transport theory and a MIL architecture to align tile score distributions between environments. By creating a "pseudo-in-domain fashion" setting for the classifier, the image classification method enables robust decision threshold/cutoff calibration with minimal calibration samples, especially in scenarios with limited prevalence. Validated across multiple external environments, such as multiple external cohorts, and pathology tasks, the above-described method demonstrates superior performance in maintaining targeted sensitivity levels, providing a practical solution for reliable multi-environment deployment of computational pathology systems. By working on score distributions at tile level, the number of samples can be increased and therefore the quality of distribution matching by several orders of magnitude. For example, it is possible to merely use up to 5 samples to achieve good results. Furthermore, an importance sampling strategy within a distribution matching procedure has been introduced, which can automatically handle distribution shifts due to different prevalence in training and calibration data sets. The image classification method may be applied to several WSI classification problems such as MSI status prediction, tumour classification, chromosomal instability prediction, survival prediction, or the like.

FIG. 9 shows a system 900 for classifying an input image obtained in a first environment according to an embodiment. The system may comprise a processing unit 910 and, optionally, a storage unit 920. The processing unit 910 may perform the steps described above with respect to the previous figures. For example, the processing unit 910 is configured to tile the input image into a set of tiles, generate first tile scores for the set of tiles using a ML model, apply a transport map to the generated first tile scores to obtain second tile scores, use the second tile scores in the ML model to generate an image score for the input image, and compare the image score to a threshold to classify the input image. As described in detail above, the transport map may indicate a mapping from a first tile score distribution to a second tile score distribution, the first tile score distribution being a distribution of tile scores of a set of images obtained in the first environment and tiled into tiles and the second tile score distribution being a distribution of tile scores of a set of images obtained in a second environment and tiled into tiles. The threshold may be with respect to the second environment.

It is noted that the above-described methods may be computer-implemented. For example, a distributed server system or distributed computer system may be used for implementing the above-described method steps.

The storage unit 920 may store the instructions and steps performed by the processing unit 910.

There is also generally considered a computer program product comprising instructions adapted for causing the processing unit 910 and/or any other control circuitry to carry out and/or control any method described herein with regard to the system, in particular when executed on the processing unit 910 and/or control circuitry. Also, there is considered a carrier medium arrangement carrying and/or storing a computer program product as described herein.

FIGs. 10 to 12 are discussed below to demonstrate empirically that the above-described image classification method effectively allows sensitivity control across different experimental settings and improves existing techniques. It is shown sensitivity can be controlled even in low data and low prevalence regimes, where less than 5 positive samples may be available in a calibration set. In this regard, the above-described image classification method can improve existing work by an order of magnitude, and can fill an unmet need for the deployment of DL CPath models in clinical care.

FIG. 10 illustrates ROC curves and sensitivity versus threshold graphs for indicating the presence or absence of MSI in a private collection FTB of 602 patients, wherein a low instability/ stability of MSI (MSI-L or MSS) is predicted for 448 patients and high instability of MSI (MSI-H) is predicted for 154 patients. Reference sign 1010 indicates a ROC curve generated for a train cohort (this would be the second environment described above), reference sign 1020 indicates a ROC curve generated for a validation cohort with no calibration (this would be the first environment described above, wherein no transport map is applied), and reference sign 1030 indicates a ROC curve generated for the validation cohort for which the transport map according to the above-described method is applied (this would be the first environment described above, wherein the transport map is applied). Reference sign 1050 indicates a target sensitivity and reference sign 1040 indicates a target specificity. Reference sign 1060 indicates the performance of a random model with AUC = 0.5.

As illustrated in FIG. 10 (a) (left side), the intervention at tile score level by applying the transport map does not impact the ability of a method for classifying an input image to rank, i.e. classify, patients correctly. FIG. 10 (b) (right side) illustrates the good transfer of the sensitivity/threshold curve from a train cohort (reference sign 1010) to a calibrated external validation cohort (reference sign 1030). On the contrary, the sensitivity/threshold curve without calibration (1020) shows that applying the threshold to an external cohort without calibration would lead to a dramatic drop of sensitivity (from 90% to 20%).

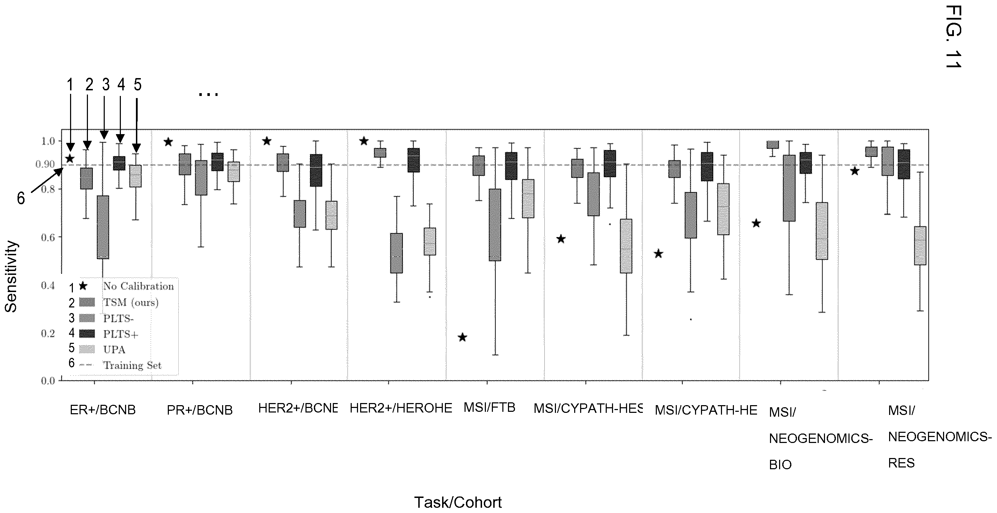

The above-described method for classifying an input image is validated against various sensitivity control methods from the prior art in FIG. 11. FIG. 11 illustrates, by way of example, a sensitivity achieved by several methods on various tasks and validation cohorts (ER+/BCNB, PR+/BCNB, HER2+/BCNB, HER2+/HEROHE, MSI/FTB, MSI/CYPATH-HES, MSI/CYPATH-HE, MSI/NEOGENOMICS-BIO, MSI/NEOGENOMICS-RES).

"BCNB" stands for a dataset with early breast cancer core-needle biopsies collected from 1,058 patients, along with clinical characteristics (227 ER- and 831 ER+, 268 PR- and 790 PR+, 781 HER2- and 277 HER2+), wherein "ER" is an estrogen receptor ("ER+": ER positive status; "ER-": ER negative status), "PR" is a progesterone receptor ("PR+": PR positive status; "PR-": PR negative status), and HER2 ("HER2+": HER positive status; "HER2-": HER negative status) is a protein present in membranes of cells to control their growth. HER2 is amplified and/or over-expressed in approximately 15-20% of breast cancers. The over-expression and/or amplification of HER2 has been associated with aggressive clinical behavior but with a high probability of response to HER2 targeted therapy during and/or after chemotherapy, resulting in a significant improvement in disease-free and overall survival.

Additionally, for HER2 status prediction, the HEROHE dataset is used, which contains 360 cases (216 HER2- and 144 HER2+).

"CYPATH" is a private collection of 698 H&E (Haematoxylin and Eosin, and H&E&S (Hematoxylin and Eosin and Saffron) biopsies from 698 patients (450 MSS/MSI-L and 248 MSI-H) digitized in France. This cohort is further split into CYPATH-HE and CYPATH-HES to account for the variations in staining conditions.

"NEOGENOMICS" is a private collection of 198 biopsies and 200 resections (226 MSS/MSI-L and 172 MSI-H overall). This cohort is further split into Neogenomics-resections ("NEOGENOMICS-RES") and Neogenomics-biopsies ("NEOGENOMICS-BIO". FTB is a private collection of 602 patients (448 MSS/MSI-L and 154 MSI-H) .

The "no calibration" stars having the reference sign 1 in FIG. 11 is a baseline where a threshold from the second environment is used. For all other methods (indicated with the reference signs 2 to 5), a calibration set of 30 slides is sampled from the first environment. Here, the process is randomly repeated 100 times. The UPA with reference sign 5 refers to a UPA implementation provided by Roschewitz, M. et al.: Automatic correction of performance drift under acquisition shift in medical image classification. Nature Communications 14(1), 6608 (Oct 2023). https://doi.org/10.1038/s41467-023-42396-y. Reference sign 2 (named "TSM") refers to a image classification method as discussed with respect to the previous figures. Reference sign 3 refers to PLTS-. Reference sign 4 refers to PLTS+. It is noted that "PLTS+" may stand for patient level threshold selection using positive patients (see sign "+"), i.e. patients labelled with "1", for example, present in the calibration set. "PLTS-" may stand for patient level threshold selection using negative patients (see sign "-"), i.e. patients labelled with "0", for example, present in the calibration set. The patient level threshold selection may leverage a calibration set composed of m WSIs with constant labels and may match the threshold to a quantile of the scores computed on the calibration data. This can be used with positive samples to control sensitivity ("PLTS+") or with negative samples to control specificity ("PLTS-").

Reference sign shows the training set. The order of the reference signs is the same for each task and for reasons of better illustration, the reference signs are not repeated for each task (see "..." in FIG. 11).

In FIG. 11, the sensitivities obtained by all methodologies using 30 WSI for calibration are reported. It is observed that that TSM and PLTS+ reach the desired sensitivity while keeping relatively close to the target, while UPA falls short in this low data regime. TSM and PLTS+ have similar average sensitivity, but TSM exhibits less variability.

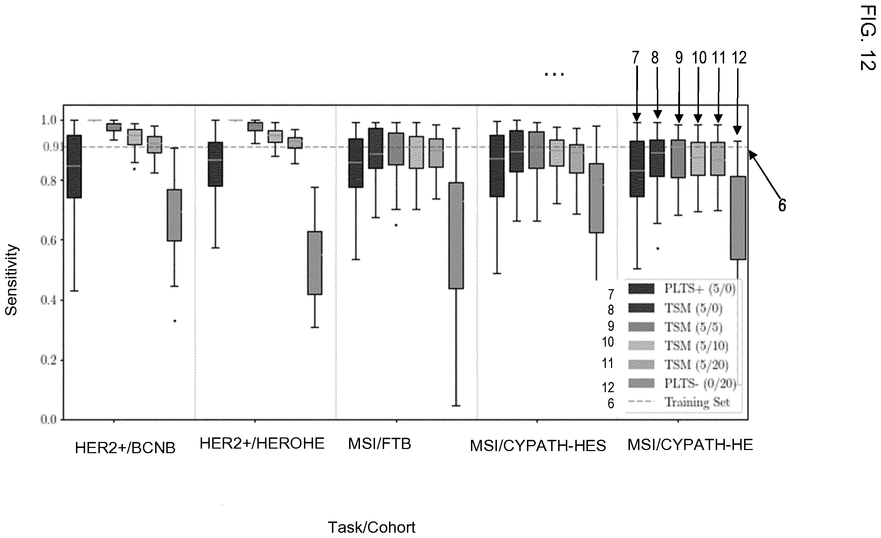

FIG. 12 illustrates, by way of example, illustrates, by way of example, a sensitivity by several methods using 5 positive samples for calibration. In FIG. 12, TSM and PLTS+ are further compared in a more challenging setting where only a handful of positive samples can be used for calibration. Here, it is focused on low prevalence tasks with less than 30% positive samples, and PLTS+ and TSM are allowed only 5 positive samples for calibration. By design, PLTS+ can only use positive samples (see reference sign 7 in FIG. 12); on the contrary, TSM is able to also leverage the negative samples (up to 20 additional negative samples, see reference signs 8 to 11 in FIG. 12). Enriched with negative samples, TSM (5/20) (reference sign 11 in FIG. 12) improves over PLTS+ (reference sign 7 in FIG. 12), both in terms of targeted sensitivity and variability.

It is noted that the ratio of positive/negative samples is provided in the legend of FIG. 12. The tasks and cohorts in FIG. 12 are equal to the tasks and cohorts in FIG. 11. The order of the reference signs is the same for each task and for reasons of better illustration, the reference signs are not repeated for each task (see "..." in FIG. 12).

Above, the transport map, such as a Monge map, has been discussed in detail. Below, an example of an implementation of the transport map is given:

As preliminaries, a probabilistic setting is first described. For example, let 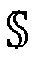 denote an observation space of a histological image, such as a WSI, *S^{t}* a histological image from a reference cohort (possibly used during training) and *Y^{t}* an associated binary label. Respectively, denote *S*^{c} a histological image from a calibration population originating from a new environment and *Y^{c}* the associated binary label. Let $\left(S\times \left\{0, 1\right\}\right.$, *S* ⊗ 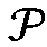 {0, 1}, µ) be a measurable space, and assume that (*S^{t},Y^{t}*) and (*S^{c},Y^{c}*) admit positive density functions with respect to a base measure µ.

Assume further that there is access to a pre-trained model $f : S ↦ ℝ$ associated to a threshold *τ* and a fixed sensitivity level *σ* such that ${ℙ}_{{S}^{t} , {Y}^{t}} \left(\left.f\left({S}^{t}\right)>τ\right|{Y}^{t}=1\right) = σ .$

This condition can be fulfilled for instance if a sufficiently large sample of a model's training data set is available to adjust the threshold *τ* to achieve the sensitivity level *σ*.

As further preliminaries, general MIL model architectures, such as a chowder model architecture (see Courtiol, P. et al.: Classification and disease localization in histopathology using only global labels: A weakly-supervised approach. arXiv preprint arXiv:1802.02212 (2018)) may be used. Such a MIL model architecture may identify a histological image $S ∈ S$ to a collection (or bag) of tiles, i.e. non-overlapping patches belonging to S. For simplicity, it is assumed that each histological image has the same number of tiles N, and denote $S = \left({T}_{1},…,{T}_{N}\right) , {T}_{i} ∈ T$ for all 1 ≤ *i* ≤ *N.* The prediction function f may be parametrized as the composition of a tile-level scoring function $g : T ↦ ℝ$, an aggregation function $r : {ℝ}^{N} ↦ {ℝ}^{2 k}$, ranking and selecting *k* - top and *k* - bottom tile scores, and a predictor $h : {ℝ}^{2 k} ↦ ℝ$: $f \left(S\right) = h \left(r\left(g\left({T}_{1}\right),…,g\left({T}_{N}\right)\right)\right) .$

Finally, a patient label may be given by $1 \left\{f\left(S\right)\geq τ\right\}$, where the threshold *τ* satisfies ${P}_{S , Y} \left(f\left(S\right)>\left.τ\right|Y=1\right) = σ ,$ and *σ* is the prescribed sensitivity level. In practice, the threshold *τ* may be adjusted in a post-training process, by leveraging a small set of held-out data, referred to as the calibration set (see, e.g. the calibration methodology deployed in Saillard, C. et al.: Validation of MSIntuit as an AI-based pre-screening tool for MSI detection from colorectal cancer histology slides. Nature Communications 14(1), 6695 (Nov 2023). https://doi.org/10.1038/s41467-023-42453-6)). As previously highlighted, *τ* often does not transfer to external cohorts (see FIG. 1), in the sense that the achieved level of sensitivity will drift apart from the prescribed target *σ* due to distributional shifts. The above-described method to mitigate this lack of threshold transferability may be applied, by matching the distribution of tile scores *g*(*Tᵢ*) between the training and application domains, using transport map (optimal transport).

As further preliminaries, tile score distribution is described. By construction and by model assumptions, for 1 ≤ *i* ≤ *n* and for *k* ∈ *{t,c},* the random variable ${T}_{i}^{k}$ may admit a positive density function on 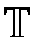 . For simplicity of exposition, it is assumed in the sequel that, in each environment, such as cohort, tiles are i.i.d. conditionally to the histological image label, i.e., for 1 ≤ *i,j* ≤ *N,k ∈* {*t*,*c*} and for *l* ∈ {0,1}: $ℙ \left(\left.{T}_{i}^{k}\right|{Y}^{k}=l\right) = ℙ \left(\left.{T}_{j}^{k}\right|{Y}^{k}=l\right) .$

This strong assumption may be required to prove a theoretical control of sensitivity. However, sensitivity may be controlled in real-life scenarios. It is assumed that the tile score function *g* is continuous. Then, the random variables corresponding to tile scores *X1 = g*(*T*1*k*)*,...,XN = g(TNk)* are also i.i.d. conditionally to the label *Y^{k}.* Denoting ${ω}^{k} = ℙ \left({Y}^{k}=\right.$ 1) the prevalence in cohort k, the density function of a tile score *X* is given for *k ∈* {*c,t*} by ${ρ}_{X}^{k} = {ω}^{k} {ρ}_{\left.X\right| Y = 1}^{k} + \left(1-{ω}^{k}\right) {ρ}_{\left.X\right| Y = 0}^{k} .$

Next, it is explained how the tile score distributions in a new environment ${ρ}_{X}^{c}$ may be matched, up to an adjustment with respect to prevalence, to the reference distribution ${ρ}_{X}^{t}$ using optimal transport.

A Monge formulation of an optimal transport problem may write, for two measures *a* and b on 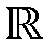 : 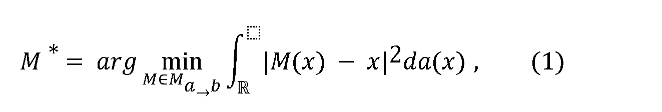 where *M*_{*a* → *b*} is a set of Borel measurable functions such that *M_{#a = b},* meaning that b is a push-forward measure of *a* through M. Since *X* is one-dimensional, this problem may have a closed-form solution, which is the monotonous map obtained through quantile matching: $M * = {F}_{b} ∘ {F}_{a}^{- 1} ,$ where *Fₐ* and *F_{b}* are the cumulative distribution functions of measures *a* and b and ${F}_{a}^{- 1}$ is the generalized inverse of *Fₐ* defined by ${F}_{a}^{- 1} \left(t\right) = inf \left\{x∈\left.ℝ\right|{F}_{a}\left(x\right)\geq t\right\} , t ∈ \left(0; 1\right) .$

The Monge formulation may be applied to $a = {ρ}_{X}^{c}$ and $b = {ω}^{c} {ρ}_{\left.X\right| Y = 1}^{t} + \left(1-{ω}^{c}\right) {ρ}_{\left.X\right| Y = 0}^{t} ,$ a reweighted version of ${ρ}_{X}^{t}$ which accounts for prevalence shift between training and validation time. It can now be proven that tile score distribution matching controls the sensitivity level in a particular case:

Theorem 1: Let $τ ∈ ℝ$, and denote by *sensₜᵣₐᵢₙ*(*τ*) and *sensᵥₐₗ*(*τ*) the calibration values associated to threshold *τ* on the train cohort and validation cohort, respectively. Assume that the calibration set contains only positive examples, i.e., *ω^{C}* = 1. Then, ${sens}_{val} \left(τ\right) = {sens}_{train} \left(τ\right) .$

Proof: After calibration, the sensitivity on the validation cohort is given by ${sens}_{val} \left(τ\right) = {ℙ}_{M * # \left({ρ}_{x}^{c}\right)} \left(\left.f\left({S}^{c}\right)>τ\right|Y=1\right) .$

However, by construction of the transport map M *, and setting*ω^{C}* = 1, $M * # \left({ρ}_{X}^{c}\right) = {ρ}_{\left.X\right| Y = 1}^{c}$ and ${ρ}_{\left.X\right| Y = 1}^{c} = {ρ}_{X}^{c}$ may be provided.

Thus, it may be obtain the following: $\begin{matrix}{sens}_{val} \left(τ\right) = {ℙ}_{M * # \left({ρ}_{X}^{c}\right)} \left(f\left({S}^{c}\right)>\left.τ\right|Y=1\right) \\ = {\int }_{{f}^{- 1} \left(\left]τ, 1\right]\right)} M * # \left({ρ}_{X}^{c}\right) \left({x}_{1}\right) … M * # \left({ρ}_{X}^{c}\right) \left({x}_{N}\right) {dx}_{1} … {dx}_{N} \\ = {\int }_{{f}^{- 1} \left(\left]τ, 1\right]\right)} {ρ}_{\left.X\right| Y = 1}^{t} \left({x}_{1}\right) … {ρ}_{\left.X\right| Y = 1}^{t} \left({x}_{N}\right) {dx}_{1} … {dx}_{N} \\ = ℙ \left({S}^{t}∈{f}^{- 1}\left.\left(\left]τ, 1\right]\right)\right|Y=1\right) \\ = ℙ \left(f\left({S}^{t}\right)>\left.τ\right|Y=1\right) \\ = {sens}_{train} \left(τ\right) .\end{matrix}$

Theorem 1 implies that applying the above-described method with a calibration set drawn from a population of a new environment conditioned on Y = 1 may effectively ensure transferability of the model's sensitivity. However, as shown in FIG. 6, using a calibration set containing both positive and negative samples can lead to similar sensitivity transfer while achieving better specificity.

Lemma 1: Let ${M}_{d}^{∗} : {R}^{d} ↦ {R}^{d}$ be the d multi-dimensional (component-wise) application of *M* *. Then ${M}_{N}^{∗} ∘ r = r ∘ {M}_{2 k}^{∗}$

Proof: The function r is a ranking function (see Courtiol, P. et al.: Deep learning-based classification of mesothelioma improves prediction of patient outcome. Nature Medicine 25(10), 1519-1525 (Oct 2019)), which associated to the monotony of *M* * conclude the proof.

Lemma 1 states that the scores coming from the same tiles will be feed in a final prediction function h, i.e. the tiles contributing to the final prediction would not change. Lemma 1 also has computational implications. Indeed, the Monge map may need to be applied only after the ranking layer r, reducing the number of computations needed.

In practice, the densities ${ρ}_{X}^{c}$ and ${ρ}_{X}^{t}$ are unknown, and instead their empirical versions may be used, defined by the weighted sum of Dirac masses centred on each data point, i.e. $\hat{{ρ}_{X}^{c}} = \frac{1}{{n}_{c}} {\sum }_{i = 1}^{{n}_{c}} {δ}_{{x}_{\left(i\right)}^{c}} , and \hat{{ρ}_{X}^{t}} = \frac{1}{{n}_{t}} {\sum }_{j = 1}^{{n}_{t}} {δ}_{{x}_{\left(j\right)}^{t}} ,$ where $\left({x}_{\left(i\right)}^{c}\right) 1 \leq i \leq {n}_{c}$ and $\left({x}_{\left(j\right)}^{t}\right) 1 \leq j \leq {n}_{t}$ are the empirical tile scores obtained by applying the tile-level score function *g* to the training and validation set of tiles, respectively.

Consequently, ${F}_{\hat{{ρ}_{X}^{c}}}$ and ${F}_{\hat{{ρ}_{X}^{t}}}$ may be staircase functions. To provide more flexibility in the mapping of points through *M* *, it may be resorted to a linear interpolation between function jumps. It will be apparent to those skilled in the art that various modifications and variations can be made in the entities and methods of this invention as well as in the construction of this invention without departing from the scope or spirit of the invention.

The invention has been described in relation to particular embodiments and examples which are intended in all aspects to be illustrative rather than restrictive. Those skilled in the art will appreciate that many different combinations of hardware, software and/or firmware will be suitable for practicing the present invention.

Moreover, other implementations of the invention will be apparent to those skilled in the art from consideration of the specification and practice of the invention disclosed herein. It is intended that the specification and the examples be considered as exemplary only. To this end, it is to be understood that inventive aspects lie in less than all features of a single foregoing disclosed implementation or configuration. Thus, the true scope and spirit of the invention is indicated by the following claims.

## Claims

1. A computer-implemented method for classifying an input image obtained in a first environment, the method comprising:
tiling the input image into a set of tiles;
generating first tile scores for the set of tiles using a machine learning, ML, model;
applying a transport map to the generated first tile scores to obtain second tile scores;
using the second tile scores in the ML model to generate an image score for the input image; and
comparing the image score to a threshold to classify the input image;
wherein the transport map indicates a mapping from a first tile score distribution to a second tile score distribution, the first tile score distribution being a distribution of tile scores of a set of images obtained in the first environment and tiled into tiles and the second tile score distribution being a distribution of tile scores of a set of images obtained in a second environment and tiled into tiles; and
wherein the threshold is with respect to the second environment.

2. The method according to claim 1, wherein the transport map is a solution of an optimal transport problem.

3. The method according to claim 1 or 2, wherein
the first tile score distribution is a density plot computed from the tile scores of the set of images obtained in the first environment;
and the second tile score distribution is a density plot computed from the tile scores of the set of images obtained in the second environment.

4. The method according any one of claims 1 to 3, wherein
a number of images included in the set of images obtained in the first environment and used for the first tile score distribution is smaller than a number of images included in the set of images obtained in the second environment and used for the second tile score distribution.

5. The method according to any one of claims 1 to 4, wherein the threshold is determined in the second environment based on a pre-specified sensitivity target or a pre-specified specificity target.

6. The method according to claim 5, wherein
the threshold is determined by generating at least one labelled image score distribution, the at least one labelled image score distribution being a distribution of image scores of a set of labelled images obtained in the second environment; and
the threshold is determined using the at least one labelled image score distribution, wherein the threshold is set in the second environment so as to reach the pre-specified sensitivity target or the pre-specified specificity target.

7. The method according to any one of claims 1 to 6, wherein the second tile score distribution is obtained based on a set of unlabelled images obtained in the second environment and tiled into tiles or based on a set of labelled images obtained in the second environment and tiled into tiles.

8. The method according to claim 7, wherein:
the set of labelled images comprises a first group of images labelled with a first label and a second group of images labelled with a second label different from the first label; and
a first labelled tile score distribution and a second labelled tile score distribution are combined using a prevalence value from the first environment to obtain the second tile score distribution;
the first labelled tile score distribution being a distribution of tile scores of the first group of images tiled into tiles, and
the second labelled tile score distribution being a distribution of tile scores of the second group of images tiled into tiles.

9. The method according to any one of claims 1 to 8, wherein the image score is compared to the threshold to obtain a binary classification of the input image.

10. The method according to claim 9, wherein the binary classification indicates presence or absence of a biomarker, indicates presence or absence of a gene mutation, indicates presence or absence of tumour cells, indicates a patient being at high risk or at low risk of an adverse clinical outcome, or indicates a patient having a low survival rate or a high survival rate.

11. The method according to claim 9 or 10, further comprising:
outputting a binary output after comparing the image score to the threshold to obtain the binary classification; and diagnosing a patient, from whom the input image is taken, based on the binary output.

12. The method according to claim 11, wherein a diagnosis report is generated for the patient based on the binary output.

13. The method according to any one of claims 1 to 12, further comprising:
extracting, for the set of tiles, feature vectors including a plurality of features, wherein the features of the feature vectors represent local descriptors of each tile in the set of tiles;
wherein the first tile scores are generated from the feature vectors using the ML model.

14. The method according to any one of claims 1 to 13, further comprising:
selecting a subset from the second tile scores;
wherein the selected subset is used in the ML model to generate the image score.

15. The method according to claim 14, wherein the subset comprises a predetermined number of the highest second tile scores and a predetermined number of the lowest second tile scores.

16. The method according to any one of claims 1 to 13, further comprising:
selecting a subset from the first tile scores;
wherein the transport map is applied to the selected subset to obtain the second tile scores.

17. The method according to claim 16, wherein the subset comprises a predetermined number of the highest first tile scores and a predetermined number of the lowest first tile scores.

18. The method according to any one of claims 1 to 17, wherein:
the ML model is a trained ML model being trained on input-output data pairs, the input-output data pairs comprising image data as input data and corresponding image scores as output data.

19. The method according to any one of claims 1 to 17, wherein:
the ML model comprises a first sub-model, the first sub-model being a trained ML model being trained on first input-output data pairs, the first input-output data pairs comprising image data as input data and corresponding tile scores as output data; and
and the ML model comprises a second sub-model, the second sub-model being a trained ML model being trained on second input-output data pairs, the second input-output data pairs comprising tile scores as input data and corresponding image scores as output data.

20. A system for classifying an input image obtained in a first environment, the system comprising:
a processing unit configured to:
tile the input image into a set of tiles;
generate first tile scores for the set of tiles using a machine learning, ML, model;
apply a transport map to the generated first tile scores to obtain second tile scores;
use the second tile scores in the ML model to generate an image score for the input image; and
compare the image score to a threshold to classify the input image;
wherein the transport map indicates a mapping from a first tile score distribution to a second tile score distribution, the first tile score distribution being a distribution of tile scores of a set of images obtained in the first environment and tiled into tiles and the second tile score distribution being a distribution of tile scores of a set of images obtained in a second environment and tiled into tiles; and
wherein the threshold is with respect to the second environment.

21. The system according to claim 19, further configured to perform the method according to any one of claims 2 to **19.**

22. A computer program comprising instructions which, when the program is executed by a computer, cause the computer to carry out the method according to any one of claims 1 to 19.

23. A computer-readable medium comprising instructions which, when executed by a computer, cause the computer to carry out the method according to any one of claims 1 to **19.**
